Europäisches Patentamt

European Patent Office

Office européen des brevets

Publication number: **0 316 592**

**A2**

# EUROPEAN PATENT APPLICATION

(21) Application number: 88117249.8

(51) Int. Cl.⁴: **C07H 19/073 , A61K 31/70**

(22) Date of filing: 17.10.88

(30) Priority: 13.11.87 US 120623

(43) Date of publication of application:
24.05.89 Bulletin 89/21

(84) Designated Contracting States:
AT BE CH DE ES FR GB GR IT LI LU NL SE

(71) Applicant: **Stichting REGA V.Z.W.**
**Minderbroedersstraat 10**
**B-3000 Leuven(BE)**

(72) Inventor: **De Clercq, Erik Désiré Alice**
**Paul Lebrunstraat 35/7**
**B-3000 Leuven(BE)**
Inventor: **Balzarini, Jan Maria René**
**Prosper Poulletlaan 10/A3**
**B-3000 Leuven(BE)**
Inventor: **Herdewijn, Piet André Maurits Maria**
**Diestsesteenweg 232**
**B-3200 Kessel-lo(BE)**

(74) Representative: **Prins, Hendrik Willem et al**
**Octrooibureau Arnold & Siedsma**
**Sweelinckplein, 1**
**NL-2517 GK The Hague(NL)**

(54) 3'-Fluoro-2',3'-dideoxyuridine, and its therapeutic application.

(57) The novel compound 3'-fluoro-2',3'-dideoxyuridine has an antiretroviral effect against human immunodeficiency virus (HIV) and is a potential antiretroviral agent for treating AIDS and related viral diseases, including hepatitis B.

F IG 2

# 3'-Fluoro-2',3'-dideoxyuridine, and its therapeutic application

This invention relates to a novel 3'-fluoro-pyrimidine-2',3'-dideoxynucleoside, 3'-fluoro-2',3'-dideoxyuridine, and its application as a novel therapeutic agent for treating retroviral diseases. including hepatitis B, and for treating AIDS and AIDS-related diseases.

Since the identification of a retrovirus, referred to as human immunodeficiency virus (HIV), as the causative agent of the acquired immunodeficiency syndrome (AIDS), many attempts have been made to develop agents that may be useful in the prevention and/or therapy of this disease. Among the 2',3'-dideoxynucleosides, several congeners have been reported to show promising antiretroviral activity. It is demonstrated that 3'-azido-2',3'-dideoxythymidine (AzddThd) is a potent inhibitor of HIV replication and protects ATH8 lymphocytes against the cytopathic effect of the virus at a concentration of 5 to 10 $\mu$M. Moreover, AzddThd protects MT4 lymphocytes against HIV at a concentration as low as 0.02 $\mu$M. 2',3'-didehydro-2',3'-dideoxythymidine and 2',3'-dideoxythymidine completely protect MT4 cells against HIV at 0,04 $\mu$M and 1 $\mu$M respectively. 2',3'-dideoxycytidine (ddCyd) is a potent inhibitor of HIV-induced cytopathogenicity in ATH8 cells, and it was shown that 2',3'-didehydro-2',3'-dideoxycytidine (ddeCyd) had a similar antiretroviral activity as ddCyd in ATH8 cells. This observation is later confirmed for MT4 cells and for human peripheral mononuclear blood cells. Interestingly, among the purine-2',3'-dideoxynucleosides, 2',3'-dideoxyadenosine (ddAdo), 2',3'-dideoxyinosine (ddIno) and 2',3'-dideoxyguanosine (ddGuo) are equally effective in protecting ATH8 cells against the cytopathogenicity of HIV (Mitsuya, H., et al, Proc. Natl. Acad. Sci. USA 83, 1911-1915 (1986)). Moreover, in ATH8 cells, the selectivity index (the ratio of the cytotoxic dose of the effective dose) was higher for ddAdo than for any of the other purine-2',3'-dideoxyriboside tested (Mitsuya, H., et al, Broder, S., Ed., Marcel Dekker Inc., New York, pp 303-333, (1986)).

During extensive research for novel potential inhibitors of HIV, a novel 3'-fluoro-substituted pyrimidine-2',3'-dideoxynucleoside was found, namely 3'-fluoro-2',3'-dideoxyuridine (FddUrd). We found that the novel FddUrd derivative is 10-fold more potent against HIV than AzddUrd.

Hereafter the anti-retrovirus potency of FddUrd will be compared with other 3'-fluoro- and 3'-azido-substituted derivatives of 2',3'-dideoxyuridine (AzddUrd), 2',3'-dideoxy-5-ethyl-uridine (FddEtUrd, AzddEtUrd) and 2',3'-dideoxycytidine (FddCyd, AzddCyd).

All reagents used were of the highest quality obtainable.

2',3'-Dideoxycytidine (ddCyd) was obtained from Pharmacia PL-Biochemicals (Piscataway, NJ, USA). 3'-Azido-2',3'-dideoxycytidine was a kind of gift of Dr. D.G. Johns (National Cancer Institute (NCI), National Institutes of Health (NIH), Bethesda, MD, USA). The method of J.P. Horwitz et al (J. Org. Chem. 31, 205-211 (1966)) was used to synthesize 2'-3'-dideoxyuridine (ddUrd). 2',3'-Dideoxy-5-ethyluridine (ddEtUrd), 3'-azido-2',3'-dideoxy-5-ethyluridine (AzddEtUrd), 3'-fluoro-2',3'-dideoxy-5-ethyluridine (FddEtUrd), and 3'-fluoro-2',3'-dideoxycytidine (FddCyd) were synthesized as described by P. Herdewijn et al (J. Med. Chem. 30, 1270-1278 (1987)). 3'-fluoro-2',3'-dideoxyuridine has been synthesized starting from 1-(5-O-trityl-2-deoxy-$\beta$-D-threo-pentofuranosyl)uracil (Lin T.-S., et al, J. Med. Chem. 26, 544, (1983)) with diethylaminosulfur trifluoride, followed by deprotection of the 5'-O-trityl group with 80% of acetic acid at 100°C. The synthesis of 3'-azido-2',3'-dideoxyuridine (AzddUrd) is described by R.F. Schinazi et al (Abstracts of the Twenty Seventh Interscience Conference on Antimicrobial Agents and Chemotherapy, New York, 4-7 October 1987, p. 161, no. 369 (1987)).

The various types of cells used in the experiments below were grown as follows.

Raji/0 cells and Raji/TK$^-$ (a thymidine (dThd) kinasedeficient mutant cell line derived from wild-type Raji/0 cells, were grown in 75 cm$^2$ plastic tissue culture flasks (International Medical, Brussels, Belgium), in Eagle's minimal essential medium, supplemented with 10% (v/v) inactivated foetal calf serum (Gibco, Glasgow, Scotland, U.K.), 2 mM L-glutamine (Flow Laboratories, Irvine, Scotland, U.K.) and 1% THM (5 mM Tes, 7.5 mM Hepes and 5 mM (MOPS) buffer (pH 5.3). The Raji/0 and Raji/TK$^-$ cells were characterized according to J. Balzarini et al (Biochem. Pharmacol. 31, 1089-1095 (1982)). Molt/4F, CEM and H9 cells were grown in Eagle's minimal essential medium, supplemented with 10% (v/v) inactivated foetal calf serum and 2 mM L-glutamine. MT4 cells were cultivated in RPMI 1640 medium (Gibco) containing 20 mM Hepes buffer, 10% (v/v) inactivated foetal calf serum (Gibco) and 2 mM L-glutamine (Flow Laboratories).

The two types of viruses used in the experiments below, were prepared from distinct sources as follows.

HTLV-III$_B$ (designated HIV) was derived from a pool of American patients with AIDS, and obtained from the supernatant of HIV-infected H9 cell cultures (see M. Popovic et al, Science 224, 497-500 (1984)).

Moloney murine sarcoma virus (MSV) was prepared from tumors induced by in vivo infection of 10 day-old NMRI mice according to the procedure described by De Clercq et al (Proc. Soc. Exp. Biol. Med. 137, 590-594 (1971)).

Therapeutic compositions containing 3'-fluoro-2',3'-dideoxyuridine as an active ingredient for treating retroviral diseases, in particular AIDS or AIDS-related diseases, including hepatitis B in human practice, may take the form of powders, suspensions, solutions, sprays, emulsions, unguents or creams and may be used for local application, for intranasal, rectal, vaginal and also for oral or parenteral (intravenous, intradermal, intramuscular, intrathecal etc.) administration. Such compositions may be prepared by combining (e.g. mixing, dissolving etc.) the active compound of 3'-fluoro-2',3'-dideoxyuridine with pharmaceutically acceptable excipients of neutral character (such as aqueous or non-aqueous solvents, stabilizers, emulsifiers, detergents, additives), and further, if necessary with dyes and aromatizers. The concentration of the active ingredient in the therapeutic composition may vary widely between 0.1% and 100%, dependent on the mode of administration. Further, the dose of the active ingredient to be administered may vary between 0.1 mg and 100 mg per kg of body weight.

The pharmaceutical properties of FddUrd, notably its anti-retroviral action, especially against HIV, are documented in comparison with other 3'-fluoro- and 3'-azido-substituted pyrimidine-2',3'-dideoxynucleosides, by the following examples which should not be read in a restrictive sense.

In the examples reference is made to the drawings annexed, in which:

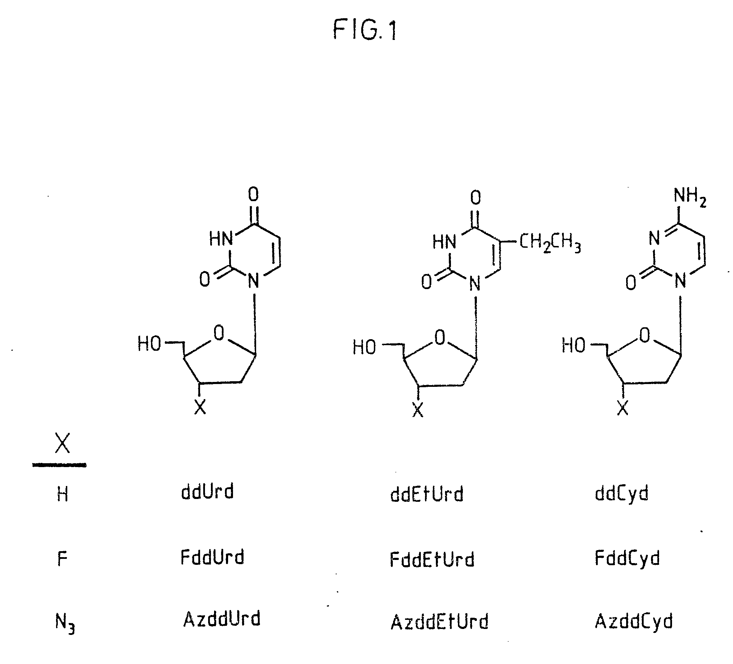

Fig. 1 shows the structural formulae of 3'-fluoro- and 3'-azido-substituted pyrimidine 2',3'-dideoxynucleosides, tested;

Fig. 2 is a representation of the test results on the inhibition of the cytopathogenicity of HIV for MT4 cells by FddUrd and AzddUrd. Viability of the cells was measured by trypan blue exclusion after an incubation period of 5 days. Mock-infected cells, incubated in the presence of different concentrations of the test compounds are indicated by open columns (□); HIV-infected cells, incubated in the presence of different concentrations of the test compounds are indicated by black columns (■). Data represent average values for 3 to 5 separate experiments;

Fig. 3 shows double-reciprocal plots for inhibition of Molt/4F dThd kinase by FddUrd (A), and AzddUrd (B). Inhibitor concentrations: none (●), 1000 $\mu$M (△) and 2000 $\mu$M (□) for FddUrd; none (●), 500 $\mu$M (△) and 1000 $\mu$M (□) for AzddUrd. Data represent average values for 3 separate experiments; and

Fig. 4 shows double-reciprocal plots for inhibition of Molt/4F dCyd kinase by FddCyd (A) and AzddCyd (B). Inhibitor concentrations: none (●), 1000 $\mu$M (□) and 200 $\mu$M (△) for FddCyd; none (●) and 250 $\mu$M (△) for AzddCyd. Data represent average values for 3 separate experiments.

## EXAMPLE 1

### Anti-retrovirus effects of FddUrd

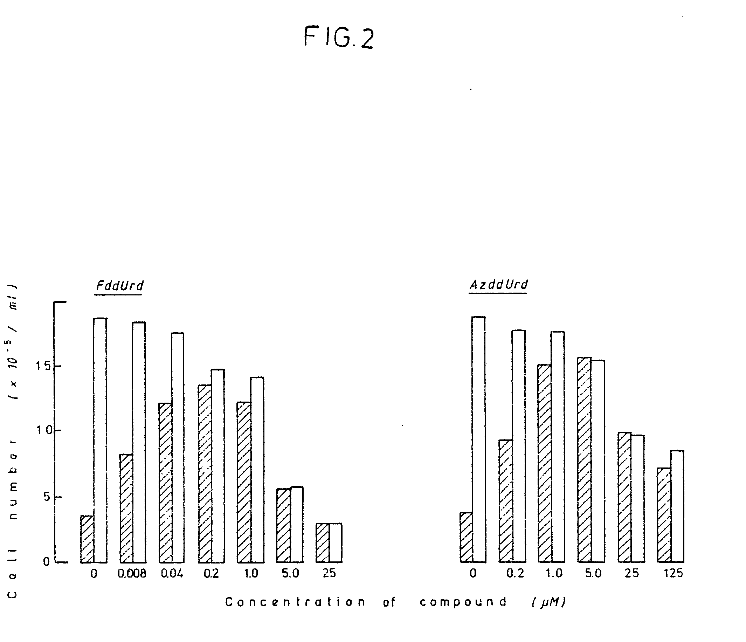

FddUrd, the unsubstituted analogue and other 3'-fluoro- and 3'-azido-substituted analogues were evaluated for their inhibitory effects on HIV-induced cytopathogenicity in MT4 cells (Table 1, fig. 2).

Human T-lymphocyte MT4 cells (5 x $10^5$ cells/ml) were suspended in fresh RPMI-1640 culture medium (Gibco) containing 10% (v/v) foetal calf serum (Gibco), 2 mM L-glutamine (Flow Laboratories), 20 mM Hepes buffer, 0.075% (w/v) NaHCO$_3$ (Flow Laboratories), 2.5 $\mu$g/ml Fungizone (Squibb N.V., Brussels, Belgium) and 20 $\mu$g/ml Geomycine (Essex N.V., Heist-o/d-Berg, Belgium), and infected with 400 CCID$_{50}$ - (Cell Culture Infective Dose-50) HIV per ml cell suspension. Then 100 $\mu$l of the infected cell suspension was added to 100 $\mu$l of an appropriate dilution of test compound in 200 $\mu$l wells of a Flat Bottom Microtest III Plate (Falcon, Becton Dickinson, Oxnard, CA, USA) (i.e. 40 CCID$_{50}$ HIV/200 $\mu$l well/5 x $10^4$ cells), and further incubated at 37° C in a CO$_2$-controlled humidified atmosphere. After incubation for 5 days, viable cell counts were determined for both virus-infected cell cultures and non-infected cell cultures (which had been incubated with the same concentration of compounds as the virus-infected cells). The 50% effective dose (ED$_{50}$) and 50% cytotoxic dose (CD$_{50}$) were defined as the compound concentrations required to reduce by 50% the number of viable cells in the virus-infected and non-infected cell cultures, respectively.

TABLE 1  ANTI-RETROVIRUS EFFECTS OF 3'-FLUORO- AND 3'-AZIDO-
SUBSTITUTED PYRIMIDINE 2',3'-DIDEOXYNUCLEOSIDE
ANALOGUES

| Compound | HIV-induced cyto-pathogenicity | | | MSV-induced cyto-pathogenicity | | |
|---|---|---|---|---|---|---|
| | $ED_{50}$(μM) 1) | $CD_{50}$(μM) 2) | S.I. 4) | $ED_{50}$(μM) 1) | $CD_{50}$(μM) 3) | S.I. 4) |
| ddUrd | 210 | >625 | > 3 | >400 | >400 | – |
| FddUrd | 0.04 | 16 | 400 | >400 | >400 | – |
| AzddUrd | 0.36 | 244 | 677 | 15 | >400 | >27 |
| ddEtUrd | >625 | >625 | – | >400 | >400 | – |
| FddEtUrd | 330 | >625 | >1.9 | >400 | >400 | – |
| AzddEtUrd | 64 | 418 | 6.5 | >200 | >200 | – |
| ddCyd | 0.3 | 40 | 120 | 25 | >400 | >16 |
| FddCyd | 16 | 26 | 1.6 | 284 | >400 | > 1.2 |
| AzddCyd | – | – | – | 70 | >400 | > 5.7 |

1) 50% antiviral effective dose, required to affect a 50%
reduction in the cytopathic effect of HIV for MT4 cells
or transformation of C3H cells by MSV.
2) 50% cytotoxic dose required to reduce the number of
viable cells in the untreated MT4 cell cultures by 50%.
3) The parameter followed here was an alteration of normal
cell morphology.
4) Selectivity index or ratio of $CD_{50}$ to $ED_{50}$.

Marked differences were noted in the anti-HIV potencies of the 3'-substituted pyrimidine 2',3'-dideoxyribosides. With an $ED_{50}$ of 0.04 μM, FddUrd was the most potent, and with an $ED_{50}$ of >64 μM, ddUrd, ddEtUrd, FddEtUrd and AzddEtUrd were the least potent HIV inhibitors.

Introduction of a 3'-azido- or 3'-fluoro-group also increased the anti-HIV potency of ddUrd. With an $ED_{50}$ of 0.04 μM FddUrd was 5000-fold and with an $ED_{50}$ of 0.36 μM AzddUrd was 600-fold more effective against HIV replication than the parent compound ddUrd ($ED_{50}$: 210 μM). Although FddUrd was 9-fold more effective against HIV than AzddUrd, it was also more cytostatic. This resulted in a selectivity index of 400 for FddUrd as compared to 677 for AzddUrd.

Among the ddCyd derivatives, 3'-fluoro-ddCyd was substantially less potent as an anti-HIV agent than ddCyd. The anti-retroviral effect of AzddCyd was not examined in the MT4 model. However, when evaluated in another T-lymphocyte cell line (ATH8), AzddCyd proved much less effective than ddCyd (H. Mitsuya et al, AIDS, Modern Concepts and Therapeutic Challenges (Broder, S., ed.), Marcel Dekker Inc., New York and Basel, pp. 303-333 (1987)).

ddUrd and the substituted pymiridine 2',3'-dideoxynucleoside analogues were examined for their inhibitory effect on the transformation of mouse embryo C3H fibroblasts by Moloney murine sarcoma virus (MSV).

C3H cells were seeded at 20,000 cells per ml into wells of Costar Tissue Culture Cluster plates (48 wells per plate) and grown to confluency. Cell cultures were then infected by 150 foci-forming units of MSV during 90 min, whereafter the culture medium was replaced by 1 ml fresh medium containing different concentrations of the test compound. After 6 days, the transformation of the cell cultures was examined microscopically.

It was surprisingly found that FddUrd was devoid of any anti-MSV activity ($ED_{50}$ >400 $\mu$M), and AzddUrd showed the highest activity ($ED_{50}$: 15 $\mu$M). Further none of the ddEtUrd derivatives had any inhibitory effect on C3H cell transformation by MSV. Among the ddCyd analogues, ddCyd had some activity ($ED_{50}$: 25 $\mu$M); the 3'-substituted ddCyd derivatives were less effective (see also table 1).

EXAMPLE 2

CYTOSTATIC AND ANTIMETABOLIC EFFECTS OF FddUrd AND 3'-SUBSTITUTED PYRIMIDINE 2',3'-DIDEOXYNUCLEOSIDE ANALOGUES

FddUrd and the other 3'-fluoro- and 3'-azido-substituted derivatives of ddUrd, ddEtUrd and ddCyd were examined for their cytostatic effects on B-lymphoblast (Raji/0), T-lymphoblast (Molt/4F) and T-lymphocyte (CEM, H9, MT4) cell lines.

All assays were performed in flat bottom Microtest III Plates (96 wells) (Falcon) as previously described by E. De Clercq et al (Mol. Pharmacol. 19, 321-330 (1981)). Briefly, the cells were suspended in growth medium and added to the microplate wells at a density of 7.5 x $10^4$ cells/well (200 $\mu$1) in the presence of varying concentrations of the test compounds. The cells were then allowed to proliferate for 68-72 hours at 37°C in a humidified, $CO_2$-controlled atmosphere. At the end of the incubation period, the cells were counted with a Coulter Counter (Coulter Electronics Ltd., Harpenden Herts, U.K.). The 50% inhibitory dose ($ID_{50}$) was defined as the concentration of compound that reduced the number of cells by 50%.

Marked differences emerged, depending on the cell line; CEM cells were 70-fold more susceptible to the cytostatic action of FddUrd ($ID_{50}$: 14 $\mu$M) than the other cell lines ($ID_{50}$ >1000 $\mu$M) (see table 2).

TABLE 2   CYTOSTATIC EFFECT OF 3'-SUBSTITUTED PYRIMIDINE 2',3'-DIDEOXYNUCLEOSIDE ANALOGUES AGAINST HUMAN B- AND T-CELL LINES

| Compound | $ID_{50}$ [1] (µM) | | | | | |
|---|---|---|---|---|---|---|
| | Raji/0 | Raji/TK⁻ | Molt/4F | CEM | H9 | MT4 |
| ddUrd | >1000 | >1000 | >1000 | >1000 | >1000 | ≥1000 |
| FddUrd | >1000 | >1000 | ≥1000 | 14 | >1000 | >1000 |
| AzddUrd | ≥1000 | >1000 | >1000 | 423 | >1000 | 364 |
| ddEtUrd | >1000 | >1000 | >1000 | >1000 | >1000 | 326 |
| FddEtUrd | >1000 | >1000 | >1000 | >1000 | >1000 | >1000 |
| AzddEtUrd | >1000 | >1000 | >1000 | >1000 | >1000 | 160 |
| ddCyd | 23 | 28 | 7 | 6 | 243 | ≥1000 |
| FddCyd | 95 | 118 | 24 | 7 | >1000 | >1000 |
| AzddCyd | – | – | – | – | – | – |

[1] 50% inhibitory dose required to reduce the cell number by 50%.

None of the 3'-substituted ddEtUrd derivatives, including ddEtUrd itself, were potent inhibitors of cell proliferation. Among the 2',3'-dideoxycytidine derivatives, ddCyd and 3'-FddCyd were more inhibitory to the proliferation of CEM and Molt/4F cells ($ID_{50}$: 6-24 µM) than Raji ($ID_{50}$: 23-95 µM), H9 and MT4 cells (243->1000 µM).

EXAMPLE 3

IMPACT ON THE CELLULAR DNA SYNTHESIS

The impact of ddUrd and the 3'-fluoro- and 3'-azido-substituted pyrimidine 2',3'-dideoxynucleoside analogues on cellular DNA biosynthesis was evaluated by monitoring the effect of these compounds on the incorporation of radio- labelled (1',2-³H)dUrd (specific radioactivity 27 Ci/mmol), (methyl-³H)dThd (specific radioactivity 480 Ci/mmol) and (5-³H)dCyd (specific radioactivity 20 Ci/mmol) into DNA of Molt/4F cells.

The incorporation of radiolabelled precursors into Molt/4F cellular DNA was measured in microtest III plates (96 wells) (Flacon) as previously described (J. Balzarini et al, Mol. Pharmacol. 23, 175-181 (1983)). Shortly, to each well of the microplate were added $10^5$ Molt/4F cells and 0.25 µCi of (1',2-³H)dUrd, (methyl³H)dThd or (5-³H)dCyd. The cells were allowed to proliferate for 20-24 hours at 37°C in a humidified $CO_2$-controlled atmosphere. At the end of this incubation period, the contents of the wells (200 µl) were brought onto 25-mm glass-fibre filters (type A/E, Gelman Instrument Company, Ann Arbor, Mich., USA) and washed twice with cold phosphate-buffered saline, twice with cold 10% trichloroacetic acid (TCA), twice with cold 5% TCA. once with ethanol and once with ether. The filters were then assayed for radioactivity in a toluene-based scintillant.

ddUrd and ddEtUrd had no effect on the incorporation of $(1',2'-{}^3H)dUrd$, $(methyl-{}^3H)dThd$ and $(5-{}^3H)-dCyd$ into Molt 4F cell DNA (Table 3).

## TABLE 3

INHIBITORY ACTIVITY OF 3'-FLUORO- AND 3'-AZIDO-SUBSTITUTED PYRIMIDINE 2',3'-DIDEOXYNUCLEOSIDES ON THE INCORPORATION OF $(1',2'-{}^3H)dUrd$, $(methyl-{}^3H)dThd$ AND $(5-{}^3H)dCyd$ INTO MOLT/4F CELL DNA

| Compound | $ID_{50}$ [1] (µM) | | |
|---|---|---|---|
| | $(1',2'-{}^3H)dUrd$ incorporation | $(methyl-{}^3H)dThd$ incorporation | $(5-{}^3H)dCyd$ incorporation |
| ddUrd | >1000 | >1000 | >1000 |
| FddUrd | 16 | >1000 | >1000 |
| AzddUrd | 172 | >1000 | 725 |
| ddEtUrd | >1000 | >1000 | >1000 |
| FddEtUrd | >1000 | >1000 | >1000 |
| AzddEtUrd | >1000 | >1000 | >1000 |
| ddCyd | 32 | 753 | >1000 (stimulation) |
| FddCyd | 27.3 | >1000 | >1000 (stimulation) |
| AzddCyd | – | – | – |

[1] 50% inhibitory dose required to reduce the incorporation of the radiolabelled precursor by 50%.

FddUrd and AzddUrd inhibited $(1',2'-{}^3H)dUrd$ incorporation into DNA to a greater extent than $(methyl-{}^3H)dThd$ or $(5-{}^3H)dCyd$ incorporation. None of the ddEtUrd derivatives showed any effect on the incorporation of the radiolabelled DNA precursors. When ddCyd and FddCyd were evaluated for their inhibitory effects on $(1',2'-{}^3H)dUrd$, $(methyl-{}^3H)dThd$ and $(5-{}^3H)dCyd$ incorporation, both compounds proved 25- to >40-fold more inhibitory to $(1',2'-{}^3H)dUrd$ incorporation than $(methyl-{}^3H)dThd$ incorporation. They significantly stimulated $(5-{}^3H)dCyd$ incorporation into DNA at the highest concentrations tested (200 µM and 1000 µM). The stimulatory effect of both ddCyd and FddCyd on $(5-{}^3H)dCyd$ incorporation into DNA amounted to 1.7-fold at 200 µM, and to 2-fold at 1000 µM, as compared to the control (data not shown).

## EXAMPLE 4

Effect of FddUrd on the inhibition of tritium release from (5-³H) dUrd or (5-³H) dCyd in Molt 4F cells

The procedure to measure tritium release from (5-³H)dUrd or (5-³H)dCyd in intact cells has been described previously (J. Balzarini et al, Biochem. Biophys. Acta 785, 36-45 (1984)). Briefly, 10⁷ Molt 4F cells/ml were preincubated with an appropriate amount of test compound for 15 min at 37° C. After this incubation period, radiolabelled (5-³H)dUrd or (5-³H)dCyd (100 µCi/ml; 0.1 µM) were added, and at various times (0, 15, 30, 45, 60 min), 100 µl of the reaction mixture was withdrawn, and mixed with 500 µl of a cold suspension of carbon black (100 mg/ml) in 5% TCA. After centrifugation at 1000 g for 10 min, the supernatants were analysed for radioactivity.

## TABLE 4

### INHIBITORY ACTIVITY OF 3'-FLUORO- AND 3'-AZIDO-SUBSTITUTED PYRIMIDINE 2',3'-DIDEOXYNUCLEOSIDES ON TRITIUM RELEASE FROM (5-³H)dUrd AND (5-³H)dCyd IN MOLT/4F CELLS

| Compound | $ID_{50}$ [1]) (µM) | |
|---|---|---|
| | (5-³H)dUrd | (5-³H)dCyd |
| ddUrd | >250 | >250 |
| FddUrd | 126 | >250 |
| AzddUrd | 20 | >250 |
| ddEtUrd | >250 | - |
| FddEtUrd | >250 | - |
| AzddEtUrd | 51 | >250 |

[1]) 50% inhibitory dose required to reduce tritium release by 50%.

None of the coumpounds evaluated affected tritium release from (5-³H)dCyd even at a concentration of 250 µM (Table 4). Among the 3'-substituted ddUrd derivatives, AzddUrd, and to a lesser extent FddUrd inhibited tritium release from (5-³H)dUrd ($ID_{50}$: 20 and 126 µM, respectively). AzddEtUrd was the only ddEtUrd derivative that inhibited tritium release from (5-³H)dUrd ($ID_{50}$: 51 µM) (Table 4.)

### EXAMPLE 5

Affinity of Molt/4F dThd kinase and dCyd kinase for FddUrd

ddUrd and the 3′-fluoro- and 3′-azido-substituted pyrimidine 2′,3′-dideoxynucleoside analogues were evaluated for their inhibitory effect against dThd and dCyd kinase.

dThd kinase and dCyd kinase were prepared from exponen tially growing Molt 4F cells, which were first washed (2x) with phosphate-buffered saline at 4° and then homogenized by sonication. The suspensions were clarified by centrifugation at 25,000-27,000 x g for 30 min. The 30-70% $(NH_4)_2SO_4$ precipitate of the supernatant was resuspended in buffer containing 10 uM potassium phosphate, pH 7.5, 10 mM $\beta$-mercaptoethanol and 0.1 M KCl, dialyzed against the same buffer, and used for the dThd kinase and the dCyd kinase assays. In these experiments, (2-$^{14}$C)dThd and (U-$^{14}$C)dCyd served as the radiolabelled substrates. The apparent $K_m$ and $K_i$ values were derived from Lineweaver-Burk plots, using a linear regression analysis program. The assay procedures have been described in detail (J. Balzarini et al, (1983) and J. Balzarini et al, Biochem. Pharmacol. 31, 3673-3682 (1982)).

TABLE 5   INHIBITION OF MOLT/4F dThd KINASE AND dCyd KINASE BY 3'-SUBSTITUTED PYRIMIDINE 2',3'-DIDEOXYNUCLEO-SIDE ANALOGUES

| Compound | Enzyme | $K_i$ | $K_i/K_m$[1]) | Type of inhibition |
|---|---|---|---|---|
| ddUrd | dThd kinase | >1000 | >250 | – |
| FddUrd | dThd kinase | 833 | 171 | competitive |
| AzddUrd | dThd kinase | 224 | 71 | competitive |
| ddEtUrd | dThd kinase | >750 | >200 | – |
| FddEtUrd | dThd kinase | 266 | 72 | competitive |
| AzddEtUrd | dThd kinase | 175 | 28 | competitive |
| ddCyd | dCyd kinase | 1965 | 404 | competitive |
| FddCyd | dCyd kinase | 360 | 63 | · competitive |
| AzddCyd | dCyd kinase | 354 | 58 | competitive |

[1]) $K_m$ values obtained in the individual experiments ranged from 2.5 to 6.6 µM for dThd kinase and 5.2 to 6.4 µM for dCyd kinase.

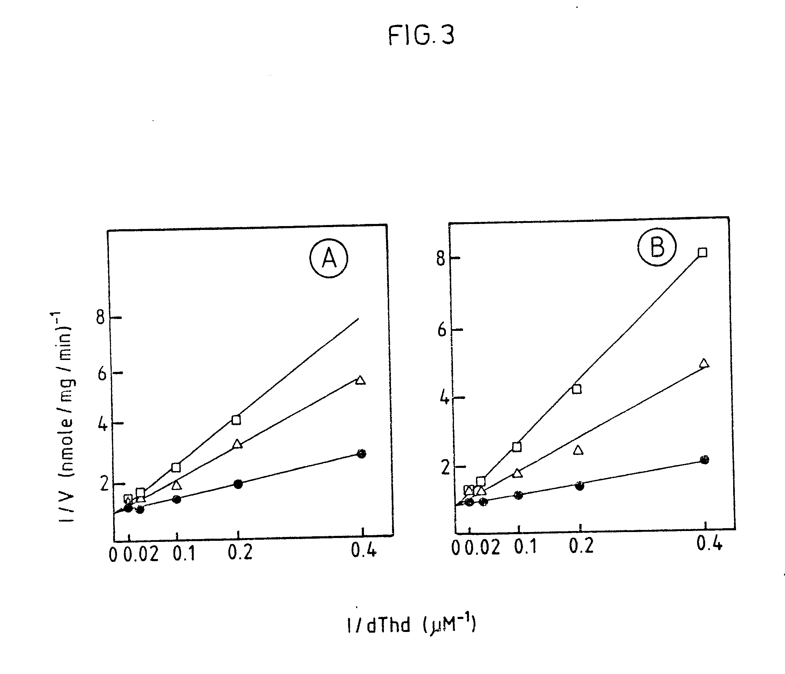

The $K_i$ and $K_i/K_m$ values of Molt/4F dThd kinase for ddUrd, ddEtUrd, and their 3′-fluoro- and 3′-azido-substituted derivatives are presented in Table 5. The unsubstituted 2′,3′-dideoxynucleosides (i.e. ddUrd and ddEtUrd) had a substantially lower affinity for dThd kinase than their 3′-fluoro- and 3′-azido-substituted counterparts. Moreover, the 3′-azido substituent conferred a greater increase in affinity of the 2′,3′-dideoxynucleoside for dThd kinase than did the 3′-fluoro substituent. The $K_i/K_m$ values for FddUrd and AzddUrd being 171 and 71, respectively. For ddUrd and ddEtUrd no inhibitory activity against dThd kinase could be detected, even when evaluated at a concentration as high as 750 µM or 1000 µM (Table 5). For those ddUrd and ddEtUrd analogues that were active as inhibitors of dThd kinase, the type of inhibition appeared o be competitive with respect to dThd. Lineweaver-Burk plots are shown for FddUrd, and AzddUrd (Fig. 3).

9

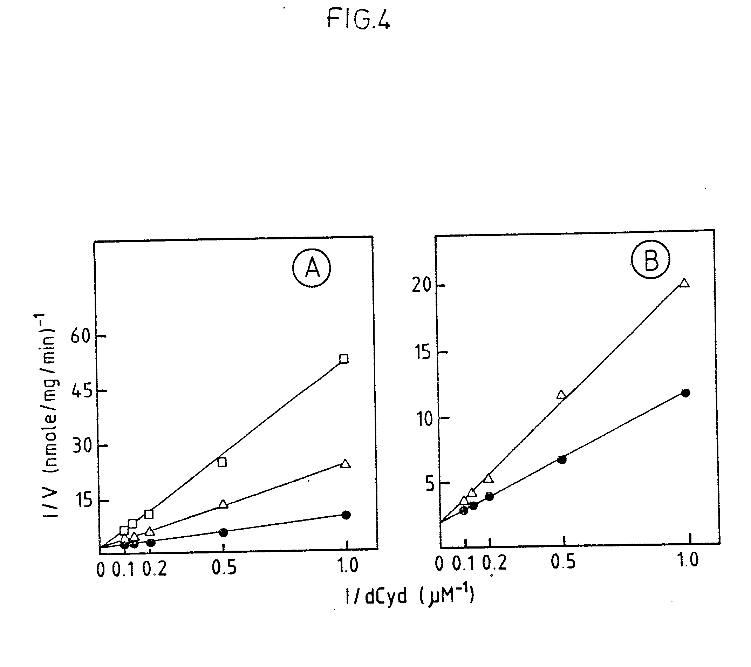

The $K_i K_m$ values of Molt 4F dCyd kinase for ddCyd, FddCyd and AzddCyd are also presented in Table 5. The $K_m$ value of the enzyme for dCyd ranged from 5.2 $\mu$M to 6.4 $\mu$M. FddCyd and AzddCyd had a relatively low affinity for the enzyme ($K_i K_m$ ~60), and for the parent nucleoside ddCyd the $K_i K_m$ value was even higher (Table 5). In all cases, inhibition was competitive with respect to radiolabelled dCyd as the competing substrate. Lineweaver-Burk plots are shown for FddCyd and AzddCyd in Fig. 4.

EXAMPLE 6

Kinetic properties of FddUrd for bacterial thymidine phosphorylase

Phosphorolytic cleavage of the test compounds was assessed using purified bacterial dThd phosphorylase (Sigma Chemical Co., St. Louis, MO) with the aid of a Beckman spectrophotometer UV Vis 5200; the absorbance change during the enzyme reaction was continuously monitored at 25°C at the wavelength where the difference of absorption between the nucleoside and its free base was maximal. The reaction mixtures for all the assays consisted of enzyme and non-satura ting concentrations of nucleoside in 0.1 M sodium phosphate buffer, pH 7.0. The initial velocity of phosphorolysis was expressed as nmol/min/enzyme unit.

The 3'-fluoro- and 3'-azido-substituted, as well as the unsubstituted, ddUrd and ddEtUrd nucleoside analogues were evaluated for their susceptibility to phosphorolysis by purified bacterial dThd phosphorylase. As compared to the 2'-deoxyribosides dUrd and dEtUrd, the 2',3'-dideoxyribosides showed a 1000- to 2000-fold decrease in the velocity of phosphorolysis by dThd phosphorylase (Table 6) (see also J. Balzarini et al, Mol. Pharmacol. 32, 162-167 (1987)). For ddEtUrd no phosphorolytic cleavage whatsoever was detectable, and this was also the case for FddEtUrd and AzddEtUrd. Introduction of a fluorine at C-3' of ddUrd further decreased the affinity for dThd phosphorylase. Introduction of an azido group at C-3' of ddUrd made the compound even totally resistant to hydrolysis by dThd phosphorylase. Similarly, ddCyd, FddCyd and AzddCyd proved totally resistant to the action of dThd phosphorylase.

TABLE 6   KINETIC PROPERTIES OF 3'-SUBSTITUTED PYRIMIDINE
          2',3'-DIDEOXYNUCLEOSIDE ANALOGUES FOR BACTERIAL
          THYMIDINE PHOSPHORYLASE

| Compound | Initial velocity of phosphorolysis (nmol/min/unit) |
|---|---|
| dUrd | 361 |
| ddUrd | 0.270 |
| AzddUrd | N.D. [1] |
| FddUrd | 0.018 |
| dEtUrd | 0.893 |
| ddEtUrd | N.D. |
| AzddEtUrd | N.D. |
| FddEtUrd | N.D. |
| dCyd | N.D. |
| ddCyd | N.D. |
| AzddCyd | N.D. |
| FddCyd | N.D. |

[1] Phosphorolysis not detectable with 25 units of enzyme.

All examples demonstrate that FddUrd, whose synthesis and biological activity has not been previously revealed, is a potent and selective anti-HIV agent and should be considered as a novel candidate compound in the treatment of retrovirus infections, for instance AIDS and AIDS-related diseases, including hepatitis B.

The introduction of a fluorine at C-3' of a pyrimidine 2',3'-dideoxynucleoside, does not inherently lead to an increase of anti-HIV activity. 3'-fluoro-substitution of ddUrd leads to an increase of anti-HIV activity, but in the case of ddCyd leads to a decrease in anti-retrovirus activi ty. Further all 3'-substituted ddEtUrd derivatives show little, if any, anti-retrovirus activity (see table 1, 5).

The differential effects of 3'-fluoro and 3'-azido substitution on the biological activity of ddUrd and ddEtUrd on the one hand, and ddCyd on the other, may be related to differences in the affinity of these compounds for the kinases involved in their phosphorylation, as well as differences in the affinity of the resulting 5'-triphosphates for the reverse transcriptase.

Claims

1. 3'-fluoro-2',3'-dideoxyuridine.

2. A therapeutic composition for use in the treatment of retroviral diseases which comprises 3'-fluoro-2',3'-dideoxyuridine as an active ingredient.

3. A therapeutic composition as claims in claim 2, comprising 3'-fluoro-2',3'-dideoxyuridine in a concentration ranging from 0.1 - 100% by weight.

4. A therapeutic composition as claimed in claim 3, having the form which is selected from the group consisting of powders, suspensions, solutions, sprays, emulsions, unguents and creams.

5. A therapeutic composition for use in the treatment of AIDS or AIDS-related diseases including hepatitis B, which comprises 3'-fluoro-2',3'-dideoxyuridine as an active ingredient.

6. A therapeutic composition as claimed in claim 5, comprising 3'-fluoro-2',3'-dideoxyuridine in a concentration ranging from 0.1 - 100% by weight.

7. A therapeutic composition as claimed in claim 6, having the form which is selected from the group consisting of powders, suspensions, solutions, sprays, emulsions, unguents and creams.

8. A method for the treatment of a retroviral disease which comprises administering 3'-fluoro-2',3'-dideoxyuridine to a patient suffering from the retroviral disease.

9. A method for the treatment of AIDS or AIDS-related diseases which comprises administering 3'-fluoro-2',3'-dideoxyuridine to a patient suffering from AIDS or AIDS-related diseases, including hepatitis B.

10. Use of 3'-fluoro-2',3'-dideoxyuridine for preparing a therapeutic composition against a retroviral disease.

11. Use of 3'-fluoro-2',3'-dideoxyuridine for preparing a therapeutic composition against AIDS or AIDS-related diseases, including hepatitis B.

# FIG.1

| X | | | |
|---|---|---|---|
| H | ddUrd | ddEtUrd | ddCyd |
| F | FddUrd | FddEtUrd | FddCyd |
| N₃ | AzddUrd | AzddEtUrd | AzddCyd |

# FIG. 2

## FIG.3

# FIG.4